# EUROPEAN PATENT APPLICATION

(11) **EP 2 281 622 A1**
(43) Date of publication of application: **09.02.2011**
(21) Application number: 10011432.1
(22) Date of filing: 12.03.2004
(51) Int. Cl.: B01D 53/88, B01D 53/22, B01J 20/02

(54) **Filtering device incorporating nanoparticles**

(30) Priority: 13.03.2003 US 387854
(62) Divisional of application: 04749379.6
(71) Applicant: Applied Nanoscience Inc., Hauppauge NY 11788 (US)
(72) Inventor: Beplate, Douglas, K., Henderson NV 89052 (US)
(74) Representative: Liesegang, Eva

(57) **Abstract**

A filtering device incorporating nanoparticles (1) that are known to be capable of destroying bacteria, fungi, viruses, or toxins. The nanoparticles (1) are combined with a filter (2). The nanoparticles (1) may be pellets adjacent to the filter (2), a powder (12) of nanoparticles (1) coating at least one side of a filter (2), or impregnated into a filter (2). Optionally, two or more filters (2) are contained within an encasement (3) having an inlet (4) and an outlet (5). Preferably, at least one filter (2) has an electrical charge that is the same as the electrical charge of at least one target particle. Also preferably, coating is accomplished by having a filter (2) to be coated carry an electrical charge that is opposite to an electrical charge carried by the nanoparticles (1) in the powder (12). Optionally, a filter (2) can be either hydrophobic or hydrophilic.

## Description

### TECHNICAL FIELD

This invention relates to a filtering device for removing biological contaminants such as bacteria, fungi, viruses, and toxins from nonaqueous fluids.

### BACKGROUND ART

A number of patents exist with devices employing both hydrophobic and hydrophilic filters. United States patent no. 6,375,854 and copending patent application serial no. 10/128,367, filed on April 22, 2002, are notable examples.

Furthermore, United States patent application 20020035032, published on March 21, 2002, discloses metal oxide and metal hydroxide nanocrystals (also termed "nanoparticles") which can be used in the form of powder or pellets for destroying bacteria, fungi, viruses, and toxins. According to that patent application, preferred metal oxides and hydroxides include MgO, CeO₂, AgO, SrO, BaO, CaO, TiO₂, ZrO₂, FeO, V₂O₃, V₂O₅, Mn₂O₃, Fe₂O₃, NiO, CuO, Al₂O₃, SiO₂, ZnO, Ag₂O, Mg(OH)₂, Ca(OH)₂, Al(OH)₃, Sr(OH)₂, Ba(OH)₂, Fe(OH)₃, Cu(OH)₃, Ni(OH)₂, Co(OH)₂, Zn(OH)₂, Ag(OH), and mixtures thereof.

That application indicates the nanoparticles can be used alone or can have at least a portion of their surfaces coated with either (a) a second metal oxide different from the first metal oxide and selected from oxides of metals selected from the group consisting of Ti, V, Fe, Cu, Ni, Co, Mn, Zn, Al, Ce, Sr, Ba, and mixtures thereof or (b) metal nitrates such as those selected from the group consisting of Cu(NO₃)₂, Ce(NO₃)₃, AgNO₃, and mixtures thereof. In a preferred embodiment, TiO₂ is coated with a mixture of cerium nitrate and copper nitrate to form [Ce(NO₃)₃--Cu(NO₃)₂]TiO₂.

Another embodiment of that application has reactive atoms stabilized on the surfaces of particulate metal oxides; such reactive atoms are different from the atoms forming the metal oxide. Again the oxides are selected from the group consisting of MgO, CeO₂, AgO, SrO, BaO, CaO, TiO₂, ZrO₂, FeO, V₂O₃, V₂O₅, Mn₂O₃, Fe₂O₃, NiO, CuO, Al₂O₃, SiO₂, ZnO, Ag₂O, and mixtures thereof. Preferably, the reactive atoms are selected from the group consisting of halogens and Group I metals. When halogens are the reactive atoms being stabilized on the surfaces of the particles, the atoms can be atoms of the same halogen, e.g., only chlorine atoms, or mixtures of atoms of different halogens, e.g., chlorine and bromine atoms.

And a final embodiment of that application has particulate metal oxides having species different from the metal oxide adsorbed on the surfaces of the metal oxide. Once more the oxides are selected from the group consisting of MgO, CeO₂, AgO, SrO, BaO, CaO, TiO₂, ZrO₂, FeO, V₂O₃, V₂O₅, Mn₂O₃, Fe₂O₃, NiO, CuO, Al₂O₃, SiO₂, ZnO, Ag₂O, and mixtures thereof. Preferably, the adsorbed species are selected from the group consisting of oxides of Group V elements, oxides of Group VI elements, and ozone. Preferred oxides of Group V and VI elements are NO₂ and SO₂, respectively.

United States patent application 20020070172, published on June 13, 2002, discloses the use of particle, pellets, and granules of fine-particle or nanoparticle iron oxides and/or iron oxyhydroxides to remove pollutants in a unit through which a fluid flows. In water purification the material is used in horizontal- or vertical-flow filters or adsorber columns or added to the water. In gas purification it is used in adsorbers for binding undesirable components such as hydrogen sulfide, mercaptans, and hydrogen cyanaide as well as other phosphorus, arsenic, antimony, sufur, selenium, tellurium, cyano, and heavy metal compounds in waste gases. Gases such as HF, HCl, H₂s, SOₓ, and NOₓ can also be adsorbed.

Finally, in June, 2002, the Subcommittee on Nanoscale Science, Engineering and Technology of the Committee on Technology for the National Science and Technology Council published the *National Nanotechnology Initiative: the Initiative and Its Implementation Plan* as a detailed technical report associated with the Supplemental Report to the President's FY 2003 Budget. This report, on pages 66 and 67, states:

"Gas mask filters used in nuclear, biological, and chemical (NBC) applications remove toxic chemicals by a process that remains essentially a WWII technology. The material responsible for chemical vapor/gas removal is an activated carbon impregnated using a Whetlerite method that impregnates metal oxides, such as, copper, zinc, molybdenum, and silver, into the larger pores of the carbon. In a very real sense activated carbon is replete with nanopores ranging from about 0.5 nm to 500 nm. Nanoscience can provide new opportunities for high surface area adsorbents and can further provide new molecular templating techniques that can augment the bonding strength. Optimized in another way, nanoporous materials can assist in the separation technologies necessary to geometrically block the migration of agents through use of a membrane.

"Collective protection systems and and protective clothing frequently utilize fibrous filters to remove agents. High-efficiency particulate arresting (HEPA) filters can be effective against particulates; even the biological toxins that might be dispersed as aerosols could be filtered out by HEPA. The use of nanotubes, nanofilaments, and nanoporous membranes might make these filters even more effective, and might include catalytic degraders as well."

None of the preceding, however, suggests using nanoparticles that are known to be capable of destroying bacteria, fungi, viruses, or toxins in conjunction with hydrophobic or hydrophilic filters. Nor, although the article seems to suggest using nanoparticles, themselves, to create a filter and may indicate impregnating carbon with nanoparticles, do the preceding seem to suggest coating any type of filter with nanoparticles, placing nanoparticle pellets adjacent to any type of filter, or impregnating any filter material other than carbon with nanoparticles.

### DISCLOSURE OF INVENTION

The present invention, in a first embodiment, combines any type of nanoparticle that is known to be capable of destroying bacteria, fungi, viruses, or toxins with one or more hydrophobic or hydrophilic filters.

The nanoparticles can be in the form of either a powder or a pellet.

When a powder is employed, the hydrophobic or hydrophilic filter is, using any technique that is known in the art, either coated or impregnated with the powder.

Preferably, in the case of coating, the hydrophobic or hydrophilic filter carries an electrostatic charge of a given polarity; and the nanoparticles are, using any technique that is well known in the art, given a charge of opposite polarity, either in the creation of the nanoparticle or through electrical induction.

In an article copyrighted by the American Chemical Society (Langmuir 2002, 18, 6679-6686) and entitled "Metal Oxide Nanoparticles as Bactericidal Agents" Peter K. Stoimenov, Rosalyn L. Klinger, George L. Marchin, and Kenneth J. Klabunde, for example, explain "... all AP-MgO/X₂ formulations are positively charged (27.0 mV (AP-MgOBr₂), 33.0 mV (AP-MgO/Cl₂), and 35.2 mV (AP-MgO) at 0.01 ionic strength NaCl)." (According to that article, "AP" indicates that the nanoparticle has been prepared through an aerogel procedure.)

When pellets are utilized, such pellets are placed adjacent to a hydrophobic or hydrophilic filter and, together with the filter, are contained within an encasement having an inlet and an outlet.

Preferably one or more hydrophobic filters are utilized in serial fluid communication with one or more hydrophilic filters. The nanoparticle coating or the pellets of nanoparticles can be placed on either the upstream or the downstream side of any one or more hydrophobic or hydrophilic filters. The filters are contained within an encasement having an inlet and an outlet, whether one or more filters is coated or has pellets adjacent to such filter or filters.

If the pellets are placed on a side of a filter which has no other filter facing it, some means for containing the pellets is necessary. In the case of the powder used to coat the filter (rather than being impregnated into the filter), a containment means is merely preferable.

For the pellets, it is preferable to have the inlet or the outlet (depending upon which is closer to the nanoparticles) of the encasement consist of one or more apertures having a maximum dimension that is less than the minimum dimension of the pellets.

For the powder coating, a membrane having a pore size smaller than the powder particles but large enough not to impede the flow of a gas substantially, preferably a pore size at least as large as the pore size of the hydrophobic or hydrophilic filter having the smallest pore size, is preferably placed across the inlet or outlet (depending upon which is closer to the nanoparticles).

Such a membrane may similarly be used when the hydrophobic or hydrophilic filter is impregnated with nanoparticles, although this is not generally done.

In further embodiments, the present invention utilizes, in place of the hydrophobic or hydrophilic filter, a filter of any type of known filter material except, in the case of impregnation with nanoparticles, carbon.

### BRIEF DESCRIPTION OF DRAWINGS

FIG. 1 portrays, in a cutaway view, nanoparticles adjacent to a filter, where the size and number of the nanoparticles has been varied for purposes of illustration.
FIG. 2 illustrates, in a cutaway view, a filter coated with nanoparticles, where the thickness of the coating has been exaggerated for purposes of illustration.
FIG. 3 shows, in a cutaway view, a filter impregnated with nanoparticles, where the size and number of the nanoparticles has been varied for purposes of illustration.
FIG. 4 depicts, in a cutaway view, an encasement having nanoparticles adjacent to and between two filters, where the size and number of the nanoparticles has been varied for purposes of illustration.
FIG. 5 is a cutaway illustration of an encasement having nanoparticles adjacent to a filter and between the filter and an inlet of the encasement, where the size and number of the nanoparticles has been varied for purposes of illustration.
FIG. 6 represents, in a cutaway view, an encasement having nanoparticles coating the side of a filter which is closer than any other side of any other filter to an inlet of the encasement, where the thickness of the coating has been exaggerated for purposes of illustration.
FIG. 7 is a cutaway view of an encasement having a filter impregnated with nanoparticles, where the size and number of the nanoparticles has been varied for purposes of illustration.

### MODES FOR CARRYING OUT THE INVENTION

As discussed above, a number of type of nanoparticles (1) are known to be capable of destroying bacteria, fungi, viruses, or toxins. The present invention combines any type of such nanoparticles (1) with one or more filters (2).

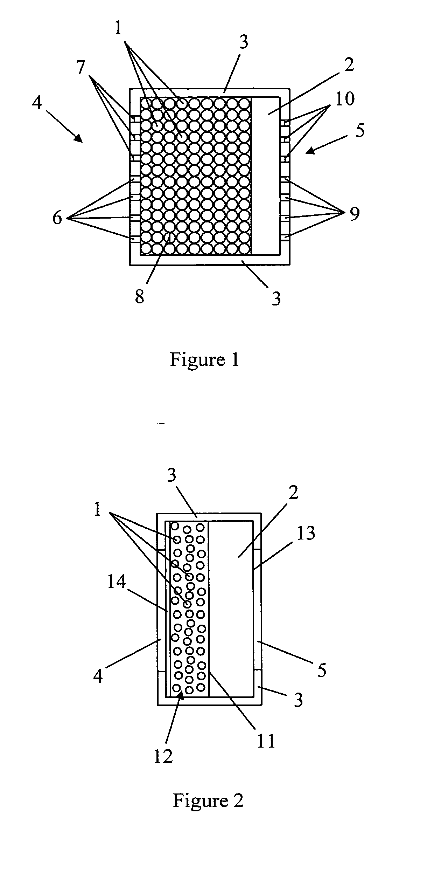

In a first principal embodiment, shown in FIG. 1, any type of nanoparticle pellets (1) that are known to be capable of destroying bacteria, fungi, viruses, or toxins are adjacent to a filter (2) within an encasement (3) having an inlet (4) and an outlet (5). Of course, if the nanoparticle pellets (1) are between the inlet (4) and the filter (2), some means must exist to contain the nanoparticle pellets (1). Any such means known in the art may be employed. Preferably, however, the one or more apertures (6) which comprise the inlet (4) each have a maximum cross-sectional dimension (7) that is less than the minimum dimension (8) of the nanoparticle pellets (1). Similarly, when the nanoparticle pellets (1) are between the outlet (5) and the filter (2), there must be a containment means, which preferably comprises having the one or more apertures (9) which comprise the outlet (5) each have a maximum dimension (10) that is less than the minimum dimension (8) of the nanoparticle pellets (1). Preferably, the nanoparticle pellets (1) are between the inlet (4) and the filter (2).

Preferably, the filter (2) has an electrical charge that is the same as the electrical charge of at least one target particle, wherein the term "target particle," as used herein, means the basic unit of any entity which the filter (2) is intended to exclude, such as a bacterium.

Optionally, the filter (2) is hydrophobic. In another optional embodiment, the filter (2) is hydrophilic.

A second principal embodiment, portrayed in FIG. 2, comprises a filter (2) coated on at least a first side (11) with a powder (12) of any type of nanoparticles (1) that are known to be capable of destroying bacteria, fungi, viruses, or toxins.

Preferably, coating is accomplished by having the filter (2) carry an electrical charge that is opposite to an electrical charge carried by the nanoparticles (1) in the powder (12). Also preferably, the filter (2) has an electrical charge that is the same as the electrical charge of at least one target particle.

Most preferably, an electrical charge on the filter (2) is both opposite to an electrical charge carried by the nanoparticles (1) in the powder (12) and the same as the electrical charge of at least one target particle. For example, the nanoparticle (1) can be AP-MgO/Br₂, AP-MgO/Cl₂, or AP-MgO, all of which are, as indicated above, positively charged. The filter (2) is then selected to have a negative electrical charge, which attracts the positively charged nanoparticles (1). Since, according to pages 6681 through 6682 in the *Langmuir* article quoted above, "... it is a well-established fact in the literature [citing Busscher, H. J.; Bos, R.; van der Mei, H. C.; Handley, P. S. in Physical Chemistry of Biological Interfaces; Baszkin, A., Norde, W., Eds.; Marcel Dekker: New York, 2000.] that the overall charge of the bacteria and spore cells at biological pH values is negative, because of the excess number of carboxylic and other groups which upon dissociation make the cell surface negative." Thus, in this most preferred situation, the electrical charge of the filter (2) tends to repel the bacteria while any bacteria that do reach the coating nanoparticle powder (12) tend to be attracted to and destroyed by the positively charged nanoparticles (1).

Again, optionally, the filter (2) can be hydrophobic; and, optionally, it can be hydrophilic. An example of a commercially available hydrophobic filter is that sold under the trademarked name FILTRETE by the 3M company of St. Paul, Minnesota. And an example of a commercially available hydrophilic filter is that sold under the name Heat and Moisture Exchange Media also by the 3M company of St. Paul, Minnesota.

Also optionally, the filter (2) is contained within an encasement (3) having an inlet (4) and an outlet (5). Preferably, the first side (11) of the filter (2) is directed toward the inlet (4) and a second side (13) of the filter (2) is directed toward the outlet (5). And preferably, if a coated side (11), (13) of the filter (2) is directed toward the inlet (4), such inlet (4) is covered by a membrane (14) having a pore size smaller than the nanoparticles (1) but large enough not to impede the flow of a gas substantially, preferably a pore size at least as large as the pore size of the filter (2). Similarly, preferably, if a coated side (11), (13) of the filter (2) is directed toward the outlet (5), such outlet (5) is covered by a membrane (14) having a pore size smaller than the nanoparticles (1) but large enough not to impede the flow of a gas substantially, preferably a pore size at least as large as the pore size of the filter (2).

Suitable membranes (14) are termed "webbing" and are, for example, commercially available from either the 3M company of St. Paul, Minnesota, or the Versal company of Los Angeles, California.

This principal embodiment was used to test the effectiveness of the nanoparticles (1) in destroying a bacterium when placed upon a hydrophobic filter (2).

### Example

A portion of a top surface of each of six horizontally oriented negatively charged hydrophobic FILTRETE filters was coated with positively charged AP-MgO/Cl₂. Also on top of the filters but not necessarily just in the location of the nanoparticles were placed an average of 226,000 colony-forming units of bacterium thuringiensis. There was no flow of air through the filter.

As a control, on a portion of a top surface of each of six uncoated horizontally oriented negatively charged hydrophobic FILTRETE filters were placed an average of 226,000 colony-forming units of bacterium thuringiensis.

After twenty-four hours, the number of colony forming units on the uncoated filters had increased by an average of more than 6507 percent while the number of colony forming units on the coated filters had decreased by an average of 21.7 percent.

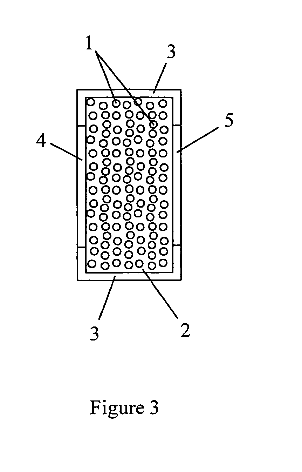

For the third principal embodiment, depicted in FIG. 3, a filter (2) is, using any technique that is known in the art, impregnated with any type of nanoparticles (1) that are known to be capable of destroying bacteria, fungi, viruses, or toxins.

Preferably, the filter (2) carries an electrical charge that is opposite to an electrical charge carried by the nanoparticles (1). Also preferably, the filter (2) has an electrical charge that is the same as the electrical charge of at least one target particle.

Most preferably, an electrical charge on the filter (2) is both opposite to an electrical charge carried by the nanoparticles (1) and the same as the electrical charge of at least one target particle.

Once again, optionally, the filter (2) can be hydrophobic; and, optionally, it can be hydrophilic.

Also optionally, the filter (2) is contained within an encasement (3) having an inlet (4) and an outlet (5).

The final four principal embodiments all employ an encasement (3) having an inlet (4) and an outlet (5) and containing two or more filters (2) in serial fluid communication with each other. Optionally, at least one of the filters (2) is hydrophobic; and, also optionally, at least one of the filters (2) is hydrophilic. Furthermore, preferably at least one of the filters (2) has an electrical charge that is the same as an electrical charge of at least one target particle; and, preferably, the filter (2) nearest the inlet (4) is hydrophobic.

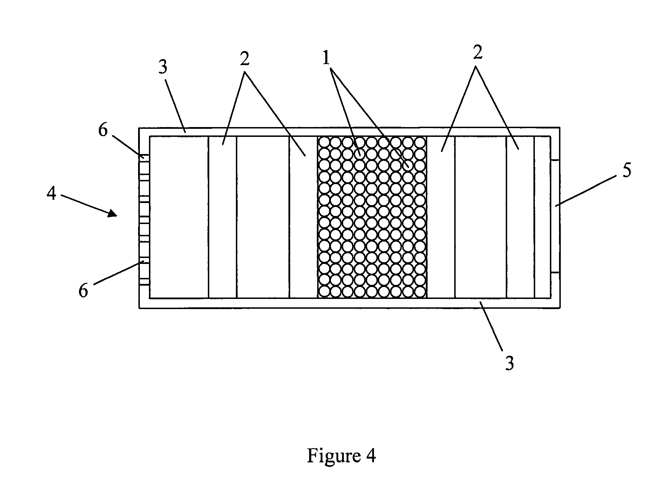

The fourth principal embodiment, illustrated in FIG. 4, has adjacent to and between at least two consecutive filters (2) any type of nanoparticle pellets (1) that are known to be capable of destroying bacteria, fungi, viruses, or toxins.

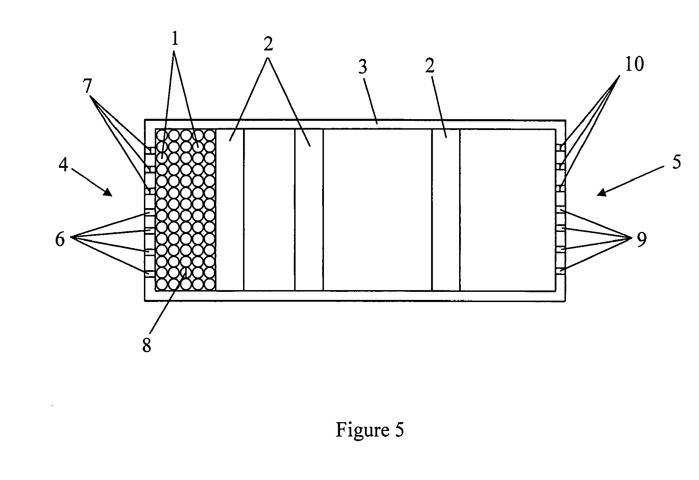

In the fifth principal embodiment, seen in FIG. 5, any type of nanoparticle pellets (1) that are known to be capable of destroying bacteria, fungi, viruses, or toxins are at least adjacent to a filter (2) that has no other filter (2) between such filter (2) and an external passageway (4), (5). As used herein, the term "external passageway" shall include both an inlet (4) and an outlet (5) and, when used in the singular, shall designate either an inlet (4) or an outlet (5). The nanoparticle pellets are between such filter (2) and the external passageway (4), (5) which is nearer to the filter (2). Preferably, such external passageway (4), (5) is the inlet (4) of the encasement (3).

Of course, as with the first principal embodiment, in the fifth principal embodiment some means must exist to contain the nanoparticle pellets (1). Any such means known in the art may be employed. Preferably, however, when the nanoparticle pellets (1) are between the filter (2) and the inlet (4), the one or more apertures (6) which comprise the inlet (4) each have a maximum dimension (7) that is less than the minimum dimension (8) of the nanoparticle pellets (1). Similarly, when the nanoparticle pellets (1) are between the outlet (5) and the filter (2), the containment means preferably comprises having the one or more apertures (9) which comprise the outlet (5) each have a maximum dimension (10) that is less than the minimum dimension (8) of the nanoparticle pellets (1).

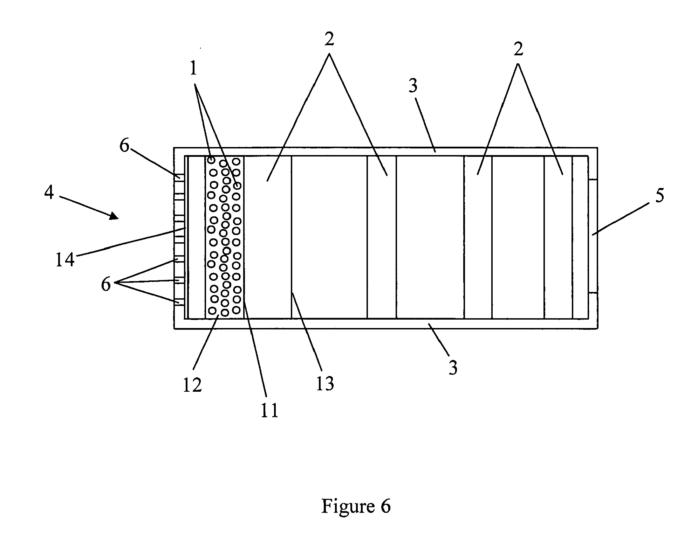

For the sixth principal embodiment, pictured in FIG. 6, a first side (11) of at least one filter (2) is coated with a powder (12) of any type of nanoparticles (1) that are known to be capable of destroying bacteria, fungi, viruses, or toxins.

Preferably, coating is accomplished by having the filter (2) carry an electrical charge that is opposite to an electrical charge carried by the nanoparticles (1) in the powder (12). Most preferably, an electrical charge on the filter (2) is both opposite to an electrical charge carried by the nanoparticles (1) and the same as the electrical charge of at least one target particle.

Also preferably, at least one such coated filter (2) has no other filter (2) between such filter (2) and the inlet (4) of the encasement (3); and most preferably the first side (11) of such filter (2) is directed toward the inlet (4).

When a coated side (11), (13) of a filter (2) is directed toward an external passageway (4), (5) and no other filter (2) is between such coated filter (2) and the external passageway (4), (5), such external passageway is preferably covered by a membrane (14) having a pore size smaller than the nanoparticles (1) but large enough not to impede the flow of a gas substantially, preferably a pore size at least as large as the pore size of the filter (2) which has the smallest pore size.

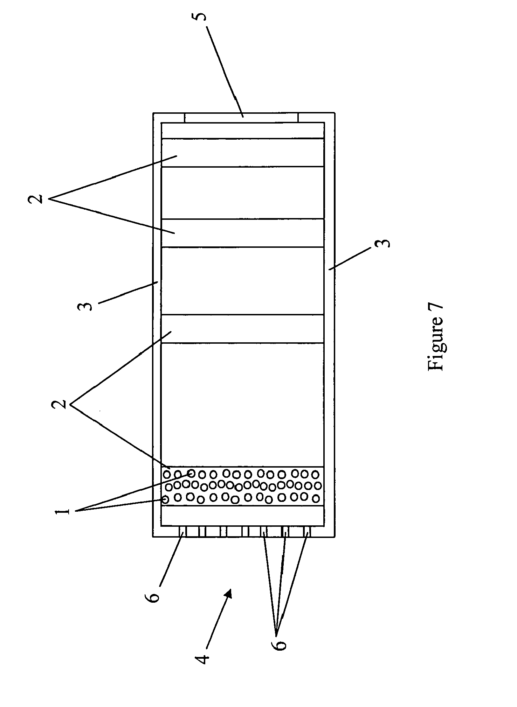

In the seventh embodiment, portrayed in FIG. 7, at least one filter (2), which is, preferably, the filter (2) closest to the inlet (4) of the encasement (3), is, using any technique that is known in the art, impregnated with any type of nanoparticles (1) that are known to be capable of destroying bacteria, fungi, viruses, or toxins.

Preferably, the impregnated filter (2) carries an electrical charge that is opposite to an electrical charge carried by the nanoparticles (1). Most preferably, an electrical charge on the impregnated filter (2) is both opposite to an electrical charge carried by the nanoparticles (1) and the same as the electrical charge of at least one target particle.

As used herein the term "preferable" or "preferably" means that a specified element or technique is more acceptable than another but not that such specified element or technique is a necessity.

### Statements

1. A filtering device incorporating nanoparticles, which comprises:
   an encasement (3) having an inlet (4) and an outlet (5);
   a filter (2) within said encasement (3);
   nanoparticle pellets (1) that are known to be capable of destroying bacteria, fungi, viruses, or toxins, said nanoparticle pellets (1) being adjacent to said filter (2) within said encasement (3); and
   a means for containing said nanoparticle pellets (1).
2. The filtering device incorporating nanoparticles as recited in statement 1, wherein:
   said filter (2) is hydrophobic.
3. The filtering device incorporating nanoparticles as recited in statement 1, wherein:
   said filter (2) is hydrophilic.
4. The filtering device incorporating nanoparticles as recited in statement 1, wherein:
   said filter (2) has an electrical charge that is the same as an electrical charge of at least one target particle.
5. The filtering device incorporating nanoparticles as recited in statement 4, wherein:
   said filter (2) is hydrophobic.
6. The filtering device incorporating nanoparticles as recited in statement 4, wherein:
   said filter (2) is hydrophilic.
7. A filtering device incorporating nanoparticles, which comprises:
   an encasement (3) having an inlet (4) and an outlet (5);
   a hydrophobic filter (2) within said encasement (3), said filter (2) having an electrical charge that is the same as an electrical charge of at least one target particle;
   nanoparticle pellets (1) that are known to be capable of destroying bacteria, fungi, viruses, or toxins, said nanoparticle pellets (1) being placed adjacent to said filter (2) within said encasement (3); and
   a means for containing said nanoparticle pellets (1).
8. A filtering device incorporating nanoparticles, which comprises:
   an encasement (3) having an inlet (4) and an outlet (5);
   a hydrophilic filter (2) within said encasement (3), said filter (2) having an electrical charge that is the same as an electrical charge of at least one target particle has;
   nanoparticle pellets (1) that are known to be capable of destroying bacteria, fungi, viruses, or toxins, said nanoparticle pellets being placed adjacent to said filter (2) within said encasement (3); and
   a means for containing said nanoparticle pellets (1).
9. A filtering device incorporating nanoparticles, which comprises:
   a filter (2) having a first side (11), a second side (13), and a pore size; and
   a powder (12) of nanoparticles (1) that are known to be capable of destroying bacteria, fungi, viruses, or toxins applied as a coating on at least the first side (11) of said filter (2).
10. The filtering device incorporating nanoparticles as recited in statement 9, wherein:
   said filter (2) is hydrophobic.
11. The filtering device incorporating nanoparticles as recited in statement 9, wherein:
   said filter (2) is hydrophilic.
12. The filtering device incorporating nanoparticles as recited in statement 9, wherein:
   the nanoparticles (1) in said powder (12) carry an electrical charge; and
   said filter (2) carries an electrical charge that is opposite to the electrical charge carried by the nanoparticles (1) in said powder (12).
13. The filtering device incorporating nanoparticles as recited in statement 12, wherein:
   said filter (2) is hydrophobic.
14. The filtering device incorporating nanoparticles as recited in statement 12, wherein:
   said filter (2) is hydrophilic.
15. The filtering device incorporating nanoparticles as recited in statement 12, wherein:
   said filter (2) has an electrical charge that is the same as an electrical charge of at least one target particle.
16. The filtering device incorporating nanoparticles as recited in statement 15, wherein:
   said filter (2) is hydrophobic.
17. The filtering device incorporating nanoparticles as recited in statement 15, wherein:
   said filter (2) is hydrophilic.
18. The filtering device incorporating nanoparticles as recited in statement 15, further comprising:
   an encasement (3) having an inlet (4) and an outlet (5), each inlet (4) and each outlet (5) constituting an external passageway and said encasement (3) containing said filter (2).
19. The filtering device incorporating nanoparticles as recited in statement 18, wherein:
   said filter is hydrophobic.
20. The filtering device incorporating nanoparticles as recited in statement 18, wherein:
   said filter is hydrophilic.
21. The filtering device incorporating nanoparticles as recited in statement 18, further comprising:
   a membrane (14) covering each external passageway toward which a side of said filter (2) that is coated with the nanoparticles (1) is directed, said membrane (14) having a pore size smaller than the nanoparticles (1) in said powder (12) but at least as large as the pore size of said filter (2).
22. The filtering device incorporating nanoparticles as recited in statement 21, wherein:
   said filter (2) is hydrophobic.
23. The filtering device incorporating nanoparticles as recited in statement 21, wherein:
   said filter (2) is hydrophilic.
24. The filtering device incorporating nanoparticles as recited in statement 15, further comprising:
   a membrane (14) covering each external passageway toward which a side of said filter (2) that is coated with the nanoparticles (1) is directed, said membrane (14) having a pore size smaller than the nanoparticles (1) in said powder but at least as large as the pore size of said filter (2).
25. The filtering device incorporating nanoparticles as recited in statement 24, wherein:
   said filter (2) is hydrophobic.
26. The filtering device incorporating nanoparticles as recited in statement 24, wherein:
   said filter (2) is hydrophilic.
27. The filtering device incorporating nanoparticles as recited in statement 12, further comprising:
   an encasement (3) having an inlet (4) and an outlet (5), each inlet (4) and each outlet (5) constituting an external passageway and said encasement (3) containing said filter (2).
28. The filtering device incorporating nanoparticles as recited in statement 27, wherein:
   said filter (2) is hydrophobic.
29. The filtering device incorporating nanoparticles as recited in statement 27, wherein:
   said filter (2) is hydrophilic.
30. The filtering device incorporating nanoparticles as recited in statement 27, further comprising:
   a membrane (14) covering each external passageway toward which a side of said filter (2) that is coated with the nanoparticles (1) is directed, said membrane (14) having a pore size smaller than the nanoparticles (1) in said powder (12) but at least as large as the pore size of said filter (2).
31. The filtering device incorporating nanoparticles as recited in statement 30, wherein:
   said filter (2) is hydrophobic.
32. The filtering device incorporating nanoparticles as recited in statement 30, wherein:
   said filter (2) is hydrophilic.
33. The filtering device incorporating nanoparticles as recited in statement 12, further comprising:
   a membrane (14) covering each external passageway toward which a side of said filter (2) that is coated with the nanoparticles (1) is directed, said membrane (14) having a pore size smaller than the nanoparticles (1) in said powder but at least as large as the pore size of said filter (2).
34. The filtering device incorporating nanoparticles as recited in statement 33, wherein:
   said filter (2) is hydrophobic.
35. The filtering device incorporating nanoparticles as recited in statement 33, wherein:
   said filter (2) is hydrophilic.
36. The filtering device incorporating nanoparticles as recited in statement 9, wherein:
   said filter (2) has an electrical charge that is the same as an electrical charge of at least one target particle.
37. The filtering device incorporating nanoparticles as recited in statement 36, wherein:
   said filter (2) is hydrophobic.
38. The filtering device incorporating nanoparticles as recited in statement 36, wherein:
   said filter (2) is hydrophilic.
39. The filtering device incorporating nanoparticles as recited in statement 36, further comprising:
   an encasement (3) having an inlet (4) and an outlet (5), each inlet (4) and each outlet (5) constituting an external passageway and said encasement (3) containing said filter (2).
40. The filtering device incorporating nanoparticles as recited in statement 39, wherein:
   said filter (2) is hydrophobic.
41. The filtering device incorporating nanoparticles as recited in statement 39, wherein:
   said filter (2) is hydrophilic.
42. The filtering device incorporating nanoparticles as recited in statement 39, further comprising:
   a membrane (14) covering each external passageway toward which a side of said filter (2) that is coated with the nanoparticles (1) is directed, said membrane (14) having a pore size smaller than the nanoparticles (1) in said powder (12) but at least as large as the pore size of said filter (2).
43. The filtering device incorporating nanoparticles as recited in statement 42, wherein:
   said filter (2) is hydrophobic.
44. The filtering device incorporating nanoparticles as recited in statement 42, wherein:
   said filter (2) is hydrophilic.
45. The filtering device incorporating nanoparticles as recited in statement 36, further comprising:
   a membrane (14) covering each external passageway toward which a side of said filter (2) that is coated with the nanoparticles (1) is directed, said membrane (14) having a pore size smaller than the nanoparticles (1) in said powder (12) but at least as large as the pore size of said filter (2).
46. The filtering device incorporating nanoparticles as recited in statement 45, wherein:
   said filter (2) is hydrophobic.
47. The filtering device incorporating nanoparticles as recited in statement 45, wherein:
   said filter (2) is hydrophilic.
48. The filtering device incorporating nanoparticles as recited in statement 9, further comprising:
   an encasement (3) having an inlet (4) and an outlet (5), each inlet (4) and each outlet (5) constituting an external passageway and said encasement (3) containing said filter (2).
49. The filtering device incorporating nanoparticles as recited in statement 48, wherein:
   said filter (2) is hydrophobic.
50. The filtering device incorporating nanoparticles as recited in statement 48, wherein:
   said filter (2) is hydrophilic.
51. The filtering device incorporating nanoparticles as recited in statement 48, further comprising:
   a membrane (14) covering each external passageway toward which a side of said filter (2) that is coated with the nanoparticles (1) is directed, said membrane having a pore size smaller than the nanoparticles (1) in said powder (12) but at least as large as the pore size of said filter (2).
52. The filtering device incorporating nanoparticles as recited in statement 51, wherein:
   said filter (2) is hydrophobic.
53. The filtering device incorporating nanoparticles as recited in statement 51, wherein:
   said filter (2) is hydrophilic.
54. The filtering device incorporating nanoparticles as recited in statement 9, further comprising:
   a membrane (14) covering each external passageway toward which a side of said filter (9) that is coated with the nanoparticles (1) is directed, said membrane (14) having a pore size smaller than the nanoparticles (1) in said powder (12) but at least as large as the pore size of said filter (2).
55. The filtering device incorporating nanoparticles as recited in statement 54, wherein:
   said filter (2) is hydrophobic.
56. The filtering device incorporating nanoparticles as recited in statement 54, wherein:
   said filter (2) is hydrophilic.
57. A filtering device incorporating nanoparticles, which comprises:
   a hydrophobic filter (2) having a first side (11), a second side (13), and a pore size, said filter (2) carrying an electrical charge that is the same as an electrical charge of at least one target particle;
   a powder (12) of any type of nanoparticles (1) that are known to be capable of destroying bacteria, fungi, viruses, or toxins applied as a coating on at least the first side (11) of said filter (2), the nanoparticles (1) of said powder (12) carrying an electrical charge that is opposite to the electrical charge carried by said filter (2);
   an encasement (3) having an inlet (4) and an outlet (5), each inlet (4) and each outlet (5) constituting an external passageway and said encasement (3) containing said filter (2); and
   a membrane (14) covering each external passageway toward which a side of said filter (2) that is coated with the nanoparticles (1) is directed, said membrane (14) having a pore size smaller than the nanoparticles (1) in said powder (12) but at least as large as the pore size of said filter (2).
58. A filtering device incorporating nanoparticles, which comprises:
   a hydrophilic filter (2) having a first side (11), a second side (13), and a pore size, said filter (2) carrying an electrical charge that is the same as an electrical charge of at least one target particle;
   a powder (12) of any type of nanoparticles (1) that are known to be capable of destroying bacteria, fungi, viruses, or toxins applied as a coating on at least the first side (11) of said filter (2), the nanoparticles (1) of said powder (12) carrying an electrical charge that is opposite to the electrical charge carried by said filter (2);
   an encasement (3) having an inlet (4) and an outlet (5), each inlet (4) and each outlet (5) constituting an external passageway and said encasement (3) containing said filter (2); and
   a membrane (14) covering each external passageway toward which a side of said filter (2) that is coated with the nanoparticles (1) is directed, said membrane (14) having a pore size smaller than the nanoparticles (1) in said powder (12) but at least as large as the pore size of said filter (2).
59. A filtering device incorporating nanoparticles, which comprises:
   a filter (2); and
   nanoparticles (1) that are known to be capable of destroying bacteria, fungi, viruses, or toxins impregnated into said filter (2).
60. The filtering device incorporating nanoparticles as recited in statement 59, wherein:
   said filter (2) is hydrophobic.
61. The filtering device incorporating nanoparticles as recited in statement 59, wherein:
   said filter (2) is hydrophilic.
62. The filtering device incorporating nanoparticles as recited in statement 59, wherein:
   said nanoparticles (1) carry an electrical charge; and
   said filter (2) carries an electrical charge that is opposite to the electrical charge carried by said nanoparticles (1).
63. The filtering device incorporating nanoparticles as recited in statement 62, wherein:
   said filter (2) is hydrophobic.
64. The filtering device incorporating nanoparticles as recited in statement 62, wherein:
   said filter (2) is hydrophilic.
65. The filtering device incorporating nanoparticles as recited in statement 62, wherein:
   said filter (2) has an electrical charge that is the same as an electrical charge of at least one target particle.
66. The filtering device incorporating nanoparticles as recited in statement 65, wherein:
   said filter (2) is hydrophobic.
67. The filtering device incorporating nanoparticles as recited in statement 65, wherein:
   said filter (2) is hydrophilic.
68. The filtering device incorporating nanoparticles as recited in statement 65, further comprising:
   an encasement (3) having an inlet (4) and an outlet (5), said encasement (3) containing said filter (2).
69. The filtering device incorporating nanoparticles as recited in statement 68, wherein:
   said filter (2) is hydrophobic.
70. The filtering device incorporating nanoparticles as recited in statement 68, wherein:
   said filter (2) is hydrophilic.
71. The filtering device incorporating nanoparticles as recited in statement 62, further comprising:
   an encasement (3) having an inlet (4) and an outlet (5), said encasement (3) containing said filter (2).
72. The filtering device incorporating nanoparticles as recited in statement 71, wherein:
   said filter (2) is hydrophobic.
73. The filtering device incorporating nanoparticles as recited in statement 71, wherein:
   said filter (2) is hydrophilic.
74. The filtering device incorporating nanoparticles as recited in statement 59, wherein:
   said filter (2) has an electrical charge that is the same as an electrical charge of at least one target particle.
75. The filtering device incorporating nanoparticles as recited in statement 74, wherein:
   said filter (2) is hydrophobic.
76. The filtering device incorporating nanoparticles as recited in statement 74, wherein:
   said filter (2) is hydrophilic.
77. The filtering device incorporating nanoparticles as recited in statement 74, further comprising:
   an encasement (3) having an inlet (4) and an outlet (5), said encasement (3) containing said filter (2).
78. The filtering device incorporating nanoparticles as recited in statement 77, wherein:
   said filter (2) is hydrophobic.
79. The filtering device incorporating nanoparticles as recited in statement 77, wherein:
   said filter (2) is hydrophilic.
80. The filtering device incorporating nanoparticles as recited in statement 59, further comprising:
   an encasement (3) having an inlet (4) and an outlet (5), said encasement (3) containing said filter (2).
81. The filtering device incorporating nanoparticles as recited in statement 80, wherein:
   said filter (2) is hydrophobic.
82. The filtering device incorporating nanoparticles as recited in statement 80, wherein:
   said filter (2) is hydrophilic.
83. A filtering device incorporating nanoparticles, which comprises:
   a hydrophobic filter (2) carrying an electrical charge that is the same as an electrical charge of at least one target particle;
   nanoparticles (1) that are known to be capable of destroying bacteria, fungi, viruses, or toxins impregnated into said filter (2), said nanoparticles (1) carrying an electrical charge that is opposite to the electrical charge carried by said filter (2); and
   an encasement (3) having an inlet (4) and an outlet (5), said encasement (3) containing said filter (2).
84. A filtering device incorporating nanoparticles, which comprises:
   a hydrophilic filter (2) carrying an electrical charge that is the same as an electrical charge of at least one target particle;
   nanoparticles (1) that are known to be capable of destroying bacteria, fungi, viruses, or toxins impregnated into said filter (2), said nanoparticles (1) carrying an electrical charge that is opposite to the electrical charge carried by said filter (2); and
   an encasement (3) having an inlet (4) and an outlet (5), said encasement (3) containing said filter (2).
85. A filtering device incorporating nanoparticles, which comprises:
   two or more filters (2) in serial fluid communication with each other;
   an encasement (3) having an inlet (4) and an outlet (5), said encasement (3) containing said filters (2); and
   nanoparticle pellets (1) that are known to be capable of destroying bacteria, fungi, viruses, or toxins adjacent to and between at least two consecutive said filters (2).
86. The filtering device incorporating nanoparticles as recited in statement 85, wherein:
   at least one of said filters (2) is hydrophobic.
87. The filtering device incorporating nanoparticles as recited in statement 85, wherein:
   at least one of said filters (2) is hydrophilic.
88. The filtering device incorporating nanoparticles as recited in statement 85, wherein:
   at least one of said filters (2) has an electrical charge that is the same as an electrical charge of at least one target particle.
89. The filtering device incorporating nanoparticles as recited in statement 88, wherein:
   at least one of said filters (2) is hydrophobic.
90. The filtering device incorporating nanoparticles as recited in statement 88, wherein:
   at least one of said filters (2) is hydrophilic.
91. The filtering device incorporating nanoparticles as recited in statement 88, wherein:
   the one of said filters (2) that is nearest to the inlet of said encasement is hydrophobic.
92. The filtering device incorporating nanoparticles as recited in statement 91, wherein:
   at least one of said filters (2) is hydrophobic.
93. The filtering device incorporating nanoparticles as recited in statement 91, wherein:
   at least one of said filters (2) is hydrophilic.
94. The filtering device incorporating nanoparticles as recited in statement 85, wherein:
   the one of said filters (2) that is nearest to the inlet (4) of said encasement (3) is hydrophobic.
95. The filtering device incorporating nanoparticles as recited in statement 94, wherein:
   at least one of said filters (2) is hydrophobic.
96. The filtering device incorporating nanoparticles as recited in statement 94, wherein:
   at least one of said filters (2) is hydrophilic.
97. A filtering device incorporating nanoparticles, which comprises:
   two or more filters (2) in serial fluid communication with each other, wherein at least one of said filters (2) has an electrical charge that is the same as an electrical charge of at least one target particle and wherein at least one of said filters (2) is hydrophobic;
   an encasement (3) having an inlet (4) and an outlet (5), wherein the one of said filters (2) that is nearest to the inlet (4) of said encasement (3) is hydrophobic; and
   nanoparticle pellets (1) that are known to be capable of destroying bacteria, fungi, viruses, or toxins adjacent to and between at least two consecutive said filters (2).
98. A filtering device incorporating nanoparticles, which comprises:
   two or more filters (2) in serial fluid communication with each other, wherein at least one of said filters (2) has an electrical charge that is the same as an electrical charge of at least one target particle and wherein at least one of said filters (2) is hydrophilic;
   an encasement (3) having an inlet (4) and an outlet (5), wherein the one of said filters (2) that is nearest to the inlet (4) of said encasement (3) is hydrophobic; and
   nanoparticle pellets (1) that are known to be capable of destroying bacteria, fungi, viruses, or toxins adjacent to and between at least two consecutive said filters (2).
99. A filtering device incorporating nanoparticles, which comprises:
   two or more filters (2) in serial fluid communication with each other;
   an encasement (3) having an inlet (4) and an outlet (5), each inlet (4) and each outlet (5) constituting an external passageway and said encasement (3) containing said filters (2);
   nanoparticle pellets (1) that are known to be capable of destroying bacteria, fungi, viruses, or toxins at least adjacent to one of said filters (2) that has no other of said filters (2) between the one of said filters (2) and an external passageway; and
   a means for containing said nanoparticle pellets (1).
100. The filtering device incorporating nanoparticles as recited in statement 99, wherein:
   at least one of said filters (2) is hydrophobic.
101. The filtering device incorporating nanoparticles as recited in statement 99, wherein:
   at least one of said filters (2) is hydrophilic.
102. The filtering device incorporating nanoparticles as recited in statement 99, wherein:
   at least one of said filters (2) has an electrical charge that is the same as an electrical charge of at least one target particle.
103. The filtering device incorporating nanoparticles as recited in statement 102, wherein:
   at least one of said filters (2) is hydrophobic.
104. The filtering device incorporating nanoparticles as recited in statement 102, wherein:
   at least one of said filters (2) is hydrophilic.
105. The filtering device incorporating nanoparticles as recited in statement 102, wherein:
   the one of said filters (2) that is nearest to the inlet (4) of said encasement (3) is hydrophobic.
106. The filtering device incorporating nanoparticles as recited in statement 105, wherein:
   at least one of said filters (2) is hydrophobic.
107. The filtering device incorporating nanoparticles as recited in statement 105, wherein:
   at least one of said filters (2) is hydrophilic.
108. The filtering device incorporating nanoparticles as recited in statement 99, wherein:
   the one of said filters (2) that is nearest to the inlet (4) of said encasement (3) is hydrophobic.
109. The filtering device incorporating nanoparticles as recited in statement 108, wherein:
   at least one of said filters (2) is hydrophobic.
110. The filtering device incorporating nanoparticles as recited in statement 108, wherein:
   at least one of said filters (2) is hydrophilic.
111. A filtering device incorporating nanoparticles, which comprises:
   two or more filters (2) in serial fluid communication with each other, wherein at least one of said filters (2) has an electrical charge that is the same as an electrical charge of at least one target particle and wherein at least one of said filters (2) is hydrophobic;
   an encasement (3) having an inlet (4) and an outlet (5), each inlet (4) and each outlet (5) constituting an external passageway and said encasement (3) containing said filters (2), wherein the one of said filters (2) that is nearest to the inlet (4) of said encasement (3) is hydrophobic;
   nanoparticle pellets (1) that are known to be capable of destroying bacteria, fungi, viruses, or toxins at least adjacent to one of said filters (2) that has no other of said filters (2) between the one of said filters (2) and an external passageway; and
   a means for containing said nanoparticle pellets (1).
112. A filtering device incorporating nanoparticles, which comprises:
   two or more filters (2) in serial fluid communication with each other, wherein at least one of said filters (2) has an electrical charge that is the same as an electrical charge of at least one target particle and wherein at least one of said filters (2) is hydrophilic;
   an encasement (3) having an inlet (4) and an outlet (5), each inlet (4) and each outlet (5) constituting an external passageway and said encasement (3) containing said filters (2), wherein the one of said filters (2) that is nearest to the inlet (4) of said encasement (3) is hydrophobic;
   nanoparticle pellets (1) that are known to be capable of destroying bacteria, fungi, viruses, or toxins at least adjacent to one of said filters (2) that has no other of said filters (2) between the one of said filters (2) and an external passageway; and
   a means for containing said nanoparticle pellets (1).
113. A filtering device incorporating nanoparticles, which comprises:
   two or more filters (2) in serial fluid communication with each other, each of said filters (2) having a first side (11), a second side (13), and a pore size;
   a powder (12) of nanoparticles (1) that are known to be capable of destroying bacteria, fungi, viruses, or toxins applied as a coating on at least the first side (11) of at least one of said filters (2); and
   an encasement (3) having an inlet (4) and an outlet (5), each inlet (4) and each outlet (5) constituting an external passageway and said encasement (3) containing said filters (2).
114. The filtering device incorporating nanoparticles as recited in statement 113, wherein:
   at least one of said filters (2) is hydrophobic.
115. The filtering device incorporating nanoparticles as recited in statement 113, wherein:
   at least one of said filters (2) is hydrophilic.
116. The filtering device incorporating nanoparticles as recited in statement 113, wherein:
   the nanoparticles (1) in said powder (12) carry an electrical charge; and
   at least one of said filters (2) that is coated with the powder (12) of nanoparticles (1) carries an electrical charge that is opposite to the electrical charge carried by the nanoparticles (1) in said powder (12).
117. The filtering device incorporating nanoparticles as recited in statement 116, wherein:
   at least one of said filters (2) is hydrophobic.
118. The filtering device incorporating nanoparticles as recited in statement 116, wherein:
   at least one of said filters (2) is hydrophilic.
119. The filtering device incorporating nanoparticles as recited in statement 116, wherein:
   at least one of said filters (2) has an electrical charge that is the same as an electrical charge of at least one target particle.
120. The filtering device incorporating nanoparticles as recited in statement 119, wherein:
   at least one of said filters (2) is hydrophobic.
121. The filtering device incorporating nanoparticles as recited in statement 119, wherein:
   at least one of said filters (2) is hydrophilic.
122. The filtering device incorporating nanoparticles as recited in statement 119, further comprising:
   a membrane (14) covering each external passageway toward which a side of one of said filters (2) that is coated with the nanoparticles (1) and that has no other of said filters (2) between the one of said filters (2) and an external passageway is directed, said membrane (14) having a pore size smaller than the nanoparticles (1) in said powder (12) but at least as large as the pore size of said filter (12) having the smallest pore size.
123. The filtering device incorporating nanoparticles as recited in statement 122, wherein:
   at least one of said filters (2) is hydrophobic.
124. The filtering device incorporating nanoparticles as recited in statement 122, wherein:
   at least one of said filters (2) is hydrophilic.
125. The filtering device incorporating nanoparticles as recited in statement 122, wherein:
   the one of said filters (2) that is nearest to the inlet (4) of said encasement (3) is hydrophobic.
126. The filtering device incorporating nanoparticles as recited in statement 125, wherein:
   at least one of said filters (2) is hydrophobic.
127. The filtering device incorporating nanoparticles as recited in statement 125, wherein:
   at least one of said filters (2) is hydrophilic.
128. The filtering device incorporating nanoparticles as recited in statement 119, wherein:
   the one of said filters (2) that is nearest to the inlet (4) of said encasement (3) is hydrophobic.
129. The filtering device incorporating nanoparticles as recited in statement 128, wherein:
   at least one of said filters (2) is hydrophobic.
130. The filtering device incorporating nanoparticles as recited in statement 128, wherein:
   at least one of said filters (2) is hydrophilic.
131. The filtering device incorporating nanoparticles as recited in statement 116, further comprising:
   a membrane (14) covering each external passageway toward which a side of one of said filters (2) that is coated with the nanoparticles (1) and that has no other of said filters (2) between the one of said filters (2) and an external passageway is directed, said membrane (14) having a pore size smaller than the nanoparticles (1) in said powder (12) but at least as large as the pore size of said filter (2) having the smallest pore size.
132. The filtering device incorporating nanoparticles as recited in statement 131, wherein:
   at least one of said filters (2) is hydrophobic.
133. The filtering device incorporating nanoparticles as recited in statement 131, wherein:
   at least one of said filters (2) is hydrophilic.
134. The filtering device incorporating nanoparticles as recited in statement 131, wherein:
   the one of said filters (2) that is nearest to the inlet (4) of said encasement (3) is hydrophobic.
135. The filtering device incorporating nanoparticles as recited in statement 134, wherein:
   at least one of said filters (2) is hydrophobic.
136. The filtering device incorporating nanoparticles as recited in statement 134, wherein:
   at least one of said filters (2) is hydrophilic.
137. The filtering device incorporating nanoparticles as recited in statement 116, wherein:
   the one of said filters (2) that is nearest to the inlet (4) of said encasement (3) is hydrophobic.
138. The filtering device incorporating nanoparticles as recited in statement 137, wherein:
   at least one of said filters (2) is hydrophobic.
139. The filtering device incorporating nanoparticles as recited in statement 137, wherein:
   at least one of said filters (2) is hydrophilic.
140. The filtering device incorporating nanoparticles as recited in statement 113, wherein:
   at least one of said filters (2) has an electrical charge that is the same as an electrical charge of at least one target particle.
141. The filtering device incorporating nanoparticles as recited in statement 140, wherein:
   at least one of said filters (2) is hydrophobic.
142. The filtering device incorporating nanoparticles as recited in statement 140, wherein:
   at least one of said filters (2) is hydrophilic.
143. The filtering device incorporating nanoparticles as recited in statement 140, further comprising:
   a membrane (14) covering each external passageway toward which a side of one of said filters (2) that is coated with the nanoparticles (1) and that has no other of said filters (2) between the one of said filters (2) and an external passageway is directed, said membrane (14) having a pore size smaller than the nanoparticles (1) in said powder (12) but at least as large as the pore size of said filter (2) having the smallest pore size.
144. The filtering device incorporating nanoparticles as recited in statement 143, wherein:
   at least one of said filters (2) is hydrophobic.
145. The filtering device incorporating nanoparticles as recited in statement 143, wherein:
   at least one of said filters (2) is hydrophilic.
146. The filtering device incorporating nanoparticles as recited in statement 143, wherein:
   the one of said filters (2) that is nearest to the inlet (4) of said encasement (3) is hydrophobic.
147. The filtering device incorporating nanoparticles as recited in statement 146, wherein:
   at least one of said filters (2) is hydrophobic.
148. The filtering device incorporating nanoparticles as recited in statement 146, wherein:
   at least one of said filters (2) is hydrophilic.
149. The filtering device incorporating nanoparticles as recited in statement 140, wherein:
   the one of said filters (2) that is nearest to the inlet (4) of said encasement (3) is hydrophobic.
150. The filtering device incorporating nanoparticles as recited in statement 149, wherein:
   at least one of said filters (2) is hydrophobic.
151. The filtering device incorporating nanoparticles as recited in statement 149, wherein:
   at least one of said filters (2) is hydrophilic.
152. The filtering device incorporating nanoparticles as recited in statement 113, further comprising:
   a membrane (14) covering each external passageway toward which a side of one of said filters (2) that is coated with the nanoparticles (1) and that has no other of said filters (2) between the one of said filters (2) and an external passageway is directed, said membrane (14) having a pore size smaller than the nanoparticles (1) in said powder (12) but at least as large as the pore size of said filter (2) having the smallest pore size.
153. The filtering device incorporating nanoparticles as recited in statement 152, wherein:
   at least one of said filters (2) is hydrophobic.
154. The filtering device incorporating nanoparticles as recited in statement 152, wherein:
   at least one of said filters (2) is hydrophilic.
155. The filtering device incorporating nanoparticles as recited in statement 152, wherein:
   the one of said filters (2) that is nearest to the inlet (4) of said encasement (3) is hydrophobic.
156. The filtering device incorporating nanoparticles as recited in statement 155, wherein:
   at least one of said filters (2) is hydrophobic.
157. The filtering device incorporating nanoparticles as recited in statement 155, wherein:
   at least one of said filters (2) is hydrophilic.
158. The filtering device incorporating nanoparticles as recited in statement 113, wherein:
   the one of said filters (2) that is nearest to the inlet (4) of said encasement (3) is hydrophobic.
159. The filtering device incorporating nanoparticles as recited in statement 158, wherein:
   at least one of said filters (2) is hydrophobic.
160. The filtering device incorporating nanoparticles as recited in statement 158, wherein:
   at least one of said filters (2) is hydrophilic.
161. A filtering device incorporating nanoparticles, which comprises:
   two or more filters (2) in serial fluid communication with each other, each of said filters (2) having a first side (11), a second side (13), and a pore size, wherein at least one of said filters (2) has an electrical charge that is the same as an electrical charge of at least one target particle and wherein at least one of said filters (2) is hydrophobic;
   a powder (12) of nanoparticles (1) that are known to be capable of destroying bacteria, fungi, viruses, or toxins applied as a coating on at least the first side (11) of at least one of said filters (2), the nanoparticles (1) in said powder (12) carrying an electrical charge that is opposite to the electrical charge carried by at least one of said filters (2) that has been coated with said powder (12);
   an encasement (3) having an inlet (4) and an outlet (5), each inlet (4) and each outlet (5) constituting an external passageway and said encasement (3) containing said filters (2), wherein the one of said filters (2) that is nearest to the inlet (4) of said encasement (3) is hydrophobic; and
   a membrane (14) covering each external passageway toward which a side of one of said filters (2) that is coated with the nanoparticles (1) and that has no other of said filters (2) between the one of said filters (2) and an external passageway is directed, said membrane (14) having a pore size smaller than the nanoparticles (1) in said powder (12) but at least as large as the pore size of said filter (2) having the smallest pore size.
162. A filtering device incorporating nanoparticles, which comprises:
   two or more filters (2) in serial fluid communication with each other, each of said filters (2) having a first side (11), a second side (13), and a pore size, wherein at least one of said filters (2) has an electrical charge that is the same as an electrical charge of at least one target particle and wherein at least one of said filters (2) is hydrophilic;
   a powder (12) of nanoparticles (1) that are known to be capable of destroying bacteria, fungi, viruses, or toxins applied as a coating on at least the first side (11) of at least one of said filters (2), the nanoparticles (1) in said powder (12) carrying an electrical charge that is opposite to the electrical charge carried by at least one of said filters (2) that has been coated with said powder (12);
   an encasement (3) having an inlet (4) and an outlet (5), each inlet (4) and each outlet (5) constituting an external passageway and said encasement (3) containing said filters (2), wherein the one of said filters (2) that is nearest to the inlet (4) of said encasement (3) is hydrophobic; and
   a membrane (14) covering each external passageway toward which a side of one of said filters (2) that is coated with the nanoparticles (1) and that has no other of said filters (2) between the one of said filters (2) and an external passageway is directed, said membrane (14) having a pore size smaller than the nanoparticles (1) in said powder (12) but at least as large as the pore size of said filter (2) having the smallest pore size.
163. A filtering device incorporating nanoparticles, which comprises:
   two or more filters (2) in serial fluid communication with each other;
   nanoparticles (1) that are known to be capable of destroying bacteria, fungi, viruses, or toxins impregnated into at least one of said filters (2); and
   an encasement (3) having an inlet (4) and an outlet (5), said encasement (3) containing said filters (2).
164. The filtering device incorporating nanoparticles as recited in statement 163, wherein:
   at least one of said filters (2) is hydrophobic.
165. The filtering device incorporating nanoparticles as recited in statement 163, wherein:
   at least one of said filters (2) is hydrophilic.
166. The filtering device incorporating nanoparticles as recited in statement 163, wherein:
   the nanoparticles (1) in said powder (12) carry an electrical charge; and
   at least one of said filters (2) that is coated with the powder (12) of nanoparticles (1) carries an electrical charge that is opposite to the electrical charge carried by the nanoparticles (1) in said powder (12).
167. The filtering device incorporating nanoparticles as recited in statement 166, wherein:
   at least one of said filters (2) is hydrophobic.
168. The filtering device incorporating nanoparticles as recited in statement 166, wherein:
   at least one of said filters is (2) hydrophilic.
169. The filtering device incorporating nanoparticles as recited in statement 166, wherein:
   at least one of said filters (2) has an electrical charge that is the same as an electrical charge of at least one target particle.
170. The filtering device incorporating nanoparticles as recited in statement 169, wherein:
   at least one of said filters (2) is hydrophobic.
171. The filtering device incorporating nanoparticles as recited in statement 169, wherein:
   at least one of said filters (2) is hydrophilic.
172. The filtering device incorporating nanoparticles as recited in statement 169, wherein:
   the one of said filters (2) that is nearest to the inlet (4) of said encasement (3) is hydrophobic.
173. The filtering device incorporating nanoparticles as recited in statement 172, wherein:
   at least one of said filters (2) is hydrophobic.
174. The filtering device incorporating nanoparticles as recited in statement 172, wherein:
   at least one of said filters (2) is hydrophilic.
175. The filtering device incorporating nanoparticles as recited in statement 166, wherein:
   the one of said filters (2) that is nearest to the inlet (4) of said encasement (3) is hydrophobic.
176. The filtering device incorporating nanoparticles as recited in statement 175, wherein:
   at least one of said filters (2) is hydrophobic.
177. The filtering device incorporating nanoparticles as recited in statement 175, wherein:
   at least one of said filters (2) is hydrophilic.
178. The filtering device incorporating nanoparticles as recited in statement 163, wherein:
   at least one of said filters (2) has an electrical charge that is the same as an electrical charge of at least one target particle.
179. The filtering device incorporating nanoparticles as recited in statement 178, wherein:
   at least one of said filters (2) is hydrophobic.
180. The filtering device incorporating nanoparticles as recited in statement 178, wherein:
   at least one of said filters (2) is hydrophilic.
181. The filtering device incorporating nanoparticles as recited in statement 178, wherein:
   the one of said filters (2) that is nearest to the inlet (4) of said encasement (3) is hydrophobic.
182. The filtering device incorporating nanoparticles as recited in statement 181, wherein:
   at least one of said filters (2) is hydrophobic.
183. The filtering device incorporating nanoparticles as recited in statement 181, wherein:
   at least one of said filters (2) is hydrophilic.
184. The filtering device incorporating nanoparticles as recited in statement 163, wherein:
   the one of said filters (2) that is nearest to the inlet (4) of said encasement (3) is hydrophobic.
185. The filtering device incorporating nanoparticles as recited in statement 184, wherein:
   at least one of said filters (2) is hydrophobic.
186. The filtering device incorporating nanoparticles as recited in statement 184, wherein:
   at least one of said filters (2) is hydrophilic.
187. A filtering device incorporating nanoparticles, which comprises:
   two or more filters (2) in serial fluid communication with each other, wherein at least one of said filters (2) has an electrical charge that is the same as an electrical charge of at least one target particle and wherein at least one of said filters (2) is hydrophobic;
   nanoparticles (1) that are known to be capable of destroying bacteria, fungi, viruses, or toxins impregnated into at least one of said filters (2), said nanoparticles (1) carrying an electrical charge that is opposite to the electrical charge carried by at least one of said filters (2); and
   an encasement (3) having an inlet (4) and an outlet (5), said encasement (3) containing said filters (2), wherein the one of said filters (2) that is nearest to the inlet (4) of said encasement (3) is hydrophobic.
188. A filtering device incorporating nanoparticles, which comprises:
   two or more filters (2) in serial fluid communication with each other, wherein at least one of said filters (2) has an electrical charge that is the same as an electrical charge of at least one target particle and wherein at least one of said filters (2) is hydrophilic;
   nanoparticles (1) that are known to be capable of destroying bacteria, fungi, viruses, or toxins impregnated into at least one of said filters (2), said nanoparticles (1) carrying an electrical charge that is opposite to the electrical charge carried by at least one of said filters (2); and
   an encasement (3) having an inlet (4) and an outlet (5), said encasement (3) containing said filters (2), wherein the one of said filters (2) that is nearest to the inlet (4) of said encasement (3) is hydrophobic.

### INDUSTRIAL APPLICABILITY

The way in which the Filtering Device Incorporating Nanoparticles is capable of exploitation in industry and the way in which the Filtering Device Incorporating Nanoparticles can be made and used are obvious from the description and the nature of the Filtering Device Incorporating Nanoparticles.

## Claims

1. A filtering device incorporating nanoparticles, which comprises:
two or more filters (2) in serial fluid communication with each other;
an encasement (3) having an inlet (4) and an outlet (5), said encasement (3) containing said filters (2); and
nanoparticle pellets (1) that are known to be capable of destroying bacteria, fungi, viruses, or toxins adjacent to and between at least two consecutive said filters (2).

2. The filtering device according to claim 1, wherein:
at least one of said filters (2) is hydrophobic.

3. The filtering device according to claim 1, wherein:
at least one of said filters (2) is hydrophilic.

4. The filtering device according to any of claims 1-3, wherein:
at least one of said filters (2) has an electrical charge that is the same as an electrical charge of at least one target particle.

5. The filtering device according to claim 4, wherein:
the one of said filters (2) that is nearest to the inlet of said encasement is hydrophobic.

6. The filtering device according to any of claims 1-3, wherein:
the one of said filters (2) that is nearest to the inlet (4) of said encasement (3) is hydrophobic.
